(19) Europäisches Patentamt / European Patent Office / Office européen des brevets

(11) **EP 3 454 822 B1**

(12) **EUROPEAN PATENT SPECIFICATION**

(45) Date of publication and mention of the grant of the patent:
**10.08.2022 Bulletin 2022/32**

(21) Application number: **17734453.8**

(22) Date of filing: **09.05.2017**

(51) International Patent Classification (IPC):
*A61K 8/24* (2006.01)   *A61Q 19/00* (2006.01)
*A61K 8/85* (2006.01)   *A61K 9/10* (2006.01)
*A61K 8/02* (2006.01)   *A61K 47/02* (2006.01)
*A61P 1/02* (2006.01)   *A61P 17/00* (2006.01)
*A61P 31/04* (2006.01)

(52) Cooperative Patent Classification (CPC):
**A61K 8/85; A61K 8/0275; A61K 8/0283;**
**A61K 8/24; A61P 1/02; A61P 17/00; A61P 31/04;**
**A61Q 19/00;** A61K 9/10; A61K 47/02

(86) International application number:
**PCT/IB2017/052696**

(87) International publication number:
**WO 2017/195108 (16.11.2017 Gazette 2017/46)**

(54) **COMPOSITION COMPRISING A POLYESTER OF BIOLOGICAL ORIGIN AND A BIOCOMPATIBLE INORGANIC COMPOUND, AND USE THEREOF IN THE COSMETIC FIELD**

ZUSAMMENSETZUNG MIT EINEM POLYESTER BIOLOGISCHEN URSPRUNGS UND EINER BIOKOMPATIBLEN ANORGANISCHEN VERBINDUNG UND VERWENDUNG DAVON IN DER KOSMETIK

COMPOSITION CONTENANT UN POLYESTER D'ORIGINE BIOLOGIQUE ET UN COMPOSÉ INORGANIQUE BIOCOMPATIBLE, ET UTILISATION DE CETTE COMPOSITION DANS LE DOMAINE COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2016 IT UA20163314**

(43) Date of publication of application:
**20.03.2019 Bulletin 2019/12**

(73) Proprietor: **BIO-ON S.p.A.**
**40016 SAN GIORGIO DI PIANO (BO) (IT)**

(72) Inventors:
• **ROVERI, Norberto**
 **40133 Bologna (IT)**
• **LELLI, Marco**
 **40063 Monghidoro (BO) (IT)**
• **MASETTI, Massimo**
 **40012 Calderara di Reno (BO) (IT)**
• **PETRAROIA, Sandra**
 **40068 San Lazzaro di Savena (BO) (IT)**

• **BEGOTTI, Simone**
 **40016 San Giorgio di Piano (BO) (IT)**

(74) Representative: **Bottero, Carlo**
**Barzanò & Zanardo Milano S.p.A.**
**Via Borgonuovo, 10**
**20121 Milano (IT)**

(56) References cited:
• **DOYLE C ET AL: "In vitro and in vivo evaluation of polyhydroxybutyrate and of polyhydroxybutyrate reinforced with hydroxyapatite", BIOMATERIALS, ELSEVIER SCIENCE PUBLISHERS BV., BARKING, GB, vol. 12, no. 9, 1 November 1991 (1991-11-01), pages 841-847, XP024141840, ISSN: 0142-9612, DOI: 10.1016/0142-9612(91)90072-I [retrieved on 1991-11-01]**

- **WEI HUANG, YINGJUN WANG, LI REN, CHANG DU, XUETAO SHI: "A novel PHBV/HA microsphere releasing system loaded with alendronate", MATERIALS SCIENCE AND ENGINEERING C, vol. 29, 30 May 2009 (2009-05-30), pages 2221-2225, XP002762861,**
- **Yingjun Wang, Xudong Wang, Kun Wei, Naru Zhao, Shuhua Zhang, Jingdi Chen: "Fabrication, characterization and long-term in vitro release ofhydrophilic drug using PHBV/HA composite microspheres", Materials Letters Material Letters, vol. 61 12 July 2006 (2006-07-12), pages 1071-1076, XP002762862, Retrieved from the Internet: URL:http://ac.els-cdn.com/S0167577X0600751 8/1-s2.0-S0167577X06007518-main.pdf?_tid=7 8b73ab8-911d-11e6-828a-00000aacb361&acdnat =1476346824_ed6995b75363f7cba7c9624297fe0 4 2a [retrieved on 2016-10-13]**

Remarks:

The file contains technical information submitted after the application was filed and not included in this specification

Remarks:

**Description**

The present invention relates to a composition comprising a polyester of a biological origin and a biocompatible inorganic compound, and its relative use in the cosmetic field. More specifically, the present invention relates to a composition comprising a polyhydroxyalkanoate and a calcium phosphate, and its relative use for conveying active ingredients in cosmetic formulations.

The use of polymeric substances is extremely widespread in the formulation of cosmetics and personal-care products. As these formulations are in most cases water-based, the polymers used must be as compatible as possible with the aqueous environment. This has in fact limited the selection to some condensation polymers, in particular polyesters and polyamides, which are generally used as fixatives for hair and encapsulants. Although these polymers have a certain instability in most aqueous environments typical of cosmetics, they have the advantage however, thanks to their low solubility in water, of not being easily removed as a result of rinsing processes and consequently remain longer on the hair and skin. This feature makes them still widely used in spite of the increasing availability of biopolymers and polyesters of a biological origin.

The use of synthesis polymers in cosmetic products and in those for personal care is an important factor in water pollution and it is consequently extremely important to substitute these polymers with other biodegradable products that do not require organic solvents and have a controllable solubility in water to allow the polymer to remain on the surface of the hair and skin as long as possible.

The biodegradability of a material is related to its susceptibility to being converted by microorganisms into simple molecules that can participate to bio-geochemical cycles. Polyhydroxyalkanoates (PHAs), polymers produced by microorganisms isolated from natural environments or also genetically modified microorganisms, are characterized by a high biodegradability. As is known, these are polymers that act as a carbon and energy reserve and which are accumulated by various species of bacteria under unfavourable growth conditions and in the presence of an excess carbon source. PHAs are synthesized and accumulated by about 300 different microbial species, included in more than 90 genera of gram-positive and gram-negative bacteria, such as, for example, *Bacillus, Rhodococcus, Rhodospirillum, Pseudomonas, Alcaligenes, Azotobacter, Rhizobium.*

In cells, the PHA is stored in the form of microgranules, whose dimension and number per cell vary in the different bacterial species. They appear under an electronic microscope as refractive inclusions, with a diameter that can range from 0.2 to 0.7 $\mu$m. Bacteria, in the absence of an extracellular carbon source, mobilize and use these reserves as carbon and energy substrates. PHAs are key compounds in regulating intracellular metabolic processes, such as for example in cellular motility and in the distribution of carbon reserves in the various metabolic pathways. Further important functions have recently been attributed to the polymer, such as cell protection against environmental stress, for example osmotic shock, UV irradiation, dehydration, thermal or oxidative stress. PHAs can be divided into two groups, depending on the number of carbon atoms forming the monomeric unit: PHAscl (short chain length) composed from 3-5 carbon atoms, PHAmcl (medium chain length) from 6-15 carbon atoms, whereas PHAlcl (long chain length) have a monomeric unit with over 15 carbon atoms. PHAscls have a high degree of crystallinity, whereas PHAmcls and PHAlcls are elastomers having a low crystallinity and with a low melting point.

The main features of PHAs are the following:

- thermoplasticity: they can be processed with various types of equipment exploiting the changes in physico-mechanical properties in relation to the temperature;
- biodegradability: this is a typical feature of polymeric compounds of a biological origin which are generally synthesized to be subsequently, if necessary, degraded again by the action of specific microbial enzymes; this characteristic makes them particularly interesting also from an environmental point of view as, thanks to their biodegradability, they do not contribute to increasing the volume of landfills, unlike conventional plastic materials;
- biocompatibility: possible applications in the medical field for the preparation of prostheses or surgical devices;

- carbon balancing: the combustion of hydrocarbons (petroleum) creates an enormous quantity of carbon dioxide which, when dispersed in the atmosphere, cannot be re-absorbed by the carbon cycle; the natural degradation of PHAs by microflora present in the ground, on the other hand, allows the cycle to be closed without dispersing carbon into the atmosphere; furthermore, biopolymers derive from renewable sources and do not depend on the availability of fossil raw materials;
- fragility and elasticity: these are both important properties of the materials; their tendency to easily break and facility in being deformed under the action of an applied force and re-acquiring their original form in the absence of this imposed action, are properties of great applicative interest;
- low permeability to gases: permeability to oxygen and permeability with respect to other gases are generally closely linked; traditional materials have a fixed relationship between permeability to oxygen and permeability to carbon dioxide; this relationship can also be observed in biopolymers, even if some of these tend to be more permeable to carbon dioxide with respect to traditional materials.

The Applicant has faced the problem of using PHAs for the formulation of cosmetic products and for personal care, which can act not only as biodegradable polymeric components that remain for a long time on the surface of the keratins of skin and hair even after prolonged rinsing, but also as innovative carriers and dispensers of specific active ingredients, whose bioactivity is prolonged after each application.

In order to achieve these results, the Applicant has found that PHA can be bound to biocompatible inorganic microparticles based on calcium phosphate, which, under physiological conditions, is able to convey the active ingredients which are used in cosmetic and personal care products, and at the same time act as a gripping agent for the PHA to prevent the same from being easily removed as a result of repeated washings.

In a first aspect, the present invention therefore relates to a composition comprising at least one polyhydroxyalkanoate (PHA) and at least one calcium phosphate, said at least one calcium phosphate being in the form of aggregates having an average size ranging from 0.1 $\mu$m to 10 $\mu$m, preferably from 0.2 $\mu$m to 5 $\mu$m, said micrometric aggregates being composed of calcium phosphate particles having average dimensions ranging from 0.01 to 1.0 microns and a crystallinity degree (CD) ranging from 50% to 80%.

As far as the PHA is concerned, this is preferably a polymer containing repeating units having formula:

$$-O\text{-}CHR_1\text{-}(CH_2)_n\text{-}CO\text{-} \qquad (I)$$

wherein:

 $R_1$ is selected from: -H, $C_1$-$C_{12}$ alkyls, $C_4$-$C_{16}$ cycloalkyls, $C_2$-$C_{12}$ alkenyls, possibly substituted by at least one group selected from: halogen (F, Cl, Br), -CN, -OH, -COOH, -OR, -COOR (R = $C_1$-$C_4$ alkyl, benzyl);
 n is zero or an integer from 1 to 6, preferably 1 or 2.

Preferably, $R_1$ is methyl or ethyl, and n is 1 or 2.

The PHAs can be either homopolymers, copolymers or terpolymers. In the case of copolymers or terpolymers, they can consist of different repeating units having formula (I), or at least one repeating unit having formula (I) combined with at least one repeating unit deriving from comonomers that are capable of copolymerizing with hydroxyalkanoates, for example lactones or lactams. In the latter case, the repeating units having formula (I) are present in a quantity equal to at least 10% by moles with respect to the total moles of repeating units.

Particularly preferred repeating units having formula (I) are those deriving from: 3-hydroxybutyrate, 3-hydroxyvalerate, 3-hydroxyhexanoate, 3-hydroxyoctanoate, 3-hydroxyundec-10-enoate, 4-hydroxyvalerate.

Particularly preferred PHAs are: poly-3-hydroxybutyrate (PHB), poly-3-hydroxyvalerate (PHV), poly-3-hydroxyhexanoate (PHH), poly-3-hydroxyoctanoate (PHO), poly(3-hydroxybutyrate-co-3-hydroxyvalerate) (PHBV), poly(3-hydroxybutyrate-co-3-hydroxyhexanoate) (PHBH), poly(3-hydroxybutyrate-co-4-hydroxybutyrate), poly(3-hydroxyoctanoate-co-3-hydroxyundec-10-enoate) (PHOU), poly(3-hydroxybutyrate-co-3-hydroxyvalerate-co-4-hydroxyvalerate (PHBVV), or mixtures thereof.

Preferably, the PHA has a weight average molecular weight ($M_w$) ranging from 5,000 to 1,500,000 Da, more preferably from 100,000 to 1,000,000 Da.

Preferably, the PHA is in the form of particles having an average size ranging from 0.1 $\mu$m to 100 $\mu$m, preferably from 0.2 $\mu$m to 20 $\mu$m.

With respect to the production of PHAs, this is preferably effected by microbial fermentation of an organic substrate (for example carbohydrates or other fermentable substrates, such as glycerol) by means of a strain of microorganisms capable of producing PHAs, and the subsequent recovery of the PHAs from the cell mass. For further details, reference could be made, for instance, to patent applications WO 99/23146, WO 2011/045625 and WO 2015/015315. Substrates suitable for the production of PHAs through fermentation can be obtained in particular from vegetable processing, for example juices, molasses, pulps deriving from processing of sugar beet or sugar cane. These substrates generally contain, in addition to sucrose and other carbohydrates, organic growth factors, nitrogen, phosphorous and/or other minerals useful as nutrients for cell growth. An alternative is glycerol, a low-cost organic carbon source, as it is a by-product of the production of biodiesel, which can possibly be used in admixture with levulinic acid (see for example patent US 8 956 835 B2).

With respect to the calcium phosphate that can be used according to the present invention, this is preferably selected from: octacalcium phosphate, tricalcium phosphate, apatite, hydroxyapatite. The calcium phosphate is more preferably apatite or hydroxyapatite.

In a preferred embodiment of the invention, the calcium phosphate also comprises zinc ions which partially substitute calcium ions. Preferably, the substitution degree of zinc ions within the calcium phosphate structure is in the range of from 0.1% to 20% by weight with respect to the total calcium content.

In another preferred embodiment of the invention, the calcium phosphate also comprises carbonate ions which partially substitute phosphate ions. Preferably, the substitution degree of the carbonate within the calcium phosphate structure is in the range of from 0.5% to 10% by weight.

In a further preferred embodiment, the hydroxyapatite has the following formula:

$$Ca_{10-x} Zn_x (PO_4)_{6-y}(CO_3)_{y+z}(OH)_{2-z}$$

wherein:

x is a number ranging from 0.0055 to 0.6;
y is a number ranging from 0.065 to 0.9; and
z is a number ranging from 0 to 0.32.

As already indicated above, the calcium phosphate is in the form of micrometric aggregates having an average size ranging from 0.1 $\mu$m to 10 $\mu$m, preferably from 0.2 $\mu$m to 5 $\mu$m. The micrometric aggregates are in turn composed of calcium phosphate particles having average dimensions ranging from 0.01 $\mu$m to 1.0 $\mu$m, preferably from 0.05 $\mu$m to 0.5 $\mu$m.

In the present description and enclosed claims, the average size of the calcium phosphate aggregates and PHA particles are determined by dynamic light scattering with laser source (DLS technique, see for example standard ISO 13320-1 (1999)). With respect to the measurement of the average size of the calcium phosphate particles, however, the TEM (transmission electron microscope) image analysis is preferred.

The calcium phosphate particles preferably have a neutralization deficiency between the positive and the negative charges on the surface, thus advantageously allowing sufficiently stable interactions to be formed (for example electrostatic bonds) with molecules and/or active ingredients of interest and for cosmetic application. In the invention, the calcium phosphate particles have a crystallinity degree (CD) ranging from 50% to 80%, more preferably ranging from 58% to 75%.

The crystallinity degree (CD) can be determined using methods well known to a skilled person in the field, such as, for example, by analysis of X-ray diffraction data. In particular, the crystallinity degree (CD) is measured according to the method described in Landi, E., Tampieri, A., Celotti, G., Sprio, S., "Densification behaviour and mechanisms of synthetic hydroxyapatites", J. Eur. Ceram. Soc., 2000, 20, 2377-2387:

$$CD = (1-X/Y) \bullet 100$$

wherein:

Y = maximum diffraction height at $2\theta = 33°$;
X = diffraction background height at $2\theta = 33°$ of the X-ray diffraction patterns of the nanoparticles.

It is believed that the calcium phosphate as defined above is capable of significantly increasing the biological activity of a biologically active molecule bound to it with respect to the activity of the same free molecule in the same biological environment. It is believed that this enhanced activity is primarily due to the distribution of free charges on the surface of the calcium phosphate particles, which spontaneously form electrostatic attractions with bioactive organic molecules. A further increase in the activity is also due to the high surface area of the particles to which the bioactive molecular is bound.

The calcium phosphate is preferably obtained by the addition of an aqueous solution of phosphoric acid ($H_3PO_4$) to an aqueous solution containing calcium ions ($Ca^{2+}$), and mixing the solution thus obtained until an aqueous suspension of calcium phosphate is obtained. The mixture is kept under stirring for a time generally ranging from 6 to 48 hours, preferably from 12 to 32 hours, at a temperature ranging from 25°C to 70°C. The suspension of calcium phosphate particles thus obtained is preferably neither washed nor filtered, but it is used as such for the preparation of the composition according to the present invention by the addition of an aqueous suspension containing at least one PHA in the form of particles in suspension. Preferably, the calcium phosphate is not calcined.

An optional active ingredient can be added during the synthesis of calcium phosphate, or to the PHA suspension, or also during the mixing of the PHA suspension with the suspension of calcium phosphate.

The preparation of the suspension of calcium phosphate as described above allows obtaining a calcium phosphate with a controlled crystallinity, within the values indicated above, since a calcium phosphate with an excessive crystallinity (higher than 90%) would significantly reduce the activity of the calcium phosphate itself, which would have a reduced capacity of binding organic molecules and active ingredients. In this respect, it should be noted that other synthesis processes of calcium phosphate would not be capable of obtaining this control of the crystallinity. In particular, the preparation of calcium phosphate in emulsion and subsequent calcination of the product after filtration would give a material with an excessive crystallinity, close to 90%, which would have a poorly reactive surface and therefore poorly suitable for being functionalized with active ingredients.

As described for example by Y. Wang et al, Materials Letters 61 (2007), 1071-1076, the synthesis of calcium phosphate (in particular hydroxyapatite) by means of an oil-in-water emulsion process comprises the preparation of a solution of dodecylamine in a mixture of ethanol and heptane, to which an aqueous solution of calcium nitrate is added under vigorous stirring. An aqueous solution of ammonium hydrogen phosphate $(NH_4)_2HPO_4$ is then added to this mixture. After keeping this mixture under stirring, the product is filtered and repeatedly washed with distilled water and ether, dried and calcined at 500°C for 28 hours. For the preparation of the complex between HA and PHBV and an active ingredient (gentamycin), an emulsion method (solid-in-oil-in-water emulsion) is again used, in which gentamycin is added to the HA nanoparticles, and the nanoparticles thus charged are mixed with a ball mill with a solution of PHA in an organic solvent (dichloromethane) with polyethyleneglycol (PEG). After pouring the mixture into water containing methylcellulose as emulsifying agent, the resulting emulsion is kept under stirring and the solvent is then evaporated. The end-product is obtained after centrifugation, filtration and drying (freeze-drying). Preferably, said at least one PHA is present in the composition according to the present invention in a quantity ranging from 60% to 98% by weight, more preferably from 75% to 95% by weight, with respect to the overall weight of the composition.

Preferably, said at least one calcium phosphate is present in the composition according to the present invention in a quantity ranging from 2% to 40% by weight, more preferably from 5% to 25% by weight, with respect to the overall weight of the composition.

It should be noted that the composition according to the present invention is stable and practically insoluble in water under neutral and basic pH conditions, whereas it is soluble in an acid environment. This feature allows the composition according to the present invention to be substantially stable under physiological pH conditions of skin and hair (neutral or slightly basic), whereas the composition rapidly dissolves under altered pH conditions (generally around 3.5-5.5, these values generally being found in the case of infections or high bacterial charge), wherein the cosmetic formulations should act to restore the physiological conditions.

Consequently, the composition according to the present invention can exert two functions when a bioactive molecule is bound to it. Under physiological pH conditions, the composition remains attached to the keratins of hair and skin even after repeated washings, gradually releasing the specific active ingredient to the same keratins also for prolonged periods of time. When, on the other hand, the composition has the function of releasing molecules having a curative effect under altered pH conditions (non-physiological), the composition is solubilized and yields the active ingredient *in situ.* An example of this application is provided by intimate soaps and oral hygiene products, which require a balancing action of the physiological pH and, in the case of oral hygiene, an opposing action to the formation of dental plaque, which is acidic.

Therefore, the composition according to the present invention preferably comprises at least one bioactive substance. This can be selected from a wide range of products, in relation to the effect to be obtained.

A first group of bioactive substances is that of antioxidants, which have the primary purpose of counteracting oxidative stress, which is a pathological condition caused by the breakdown of the physiological balance in a living organism between the production and elimination, by antioxidant defense systems, of oxidizing chemical species. Antioxidants are chemical substances (molecules, ions, radicals) or physical agents, which slow down or prevent the oxidation of other substances. Oxidation reactions can above all produce free radicals, responsible for the triggering of a chain reaction which damages the cells. Antioxidants terminate these chain reactions by intervening on the radicals and inhibiting other oxidation reactions, and consequently they essentially act as reducing substances.

Some of the antioxidants that can be used in the compositions according to the present invention are, for example:

vitamin C (ascorbic acid, ascorbate);
vitamin E (tocopherols, tocotrienols);
polyphenols (for example resveratrol, flavonoids, procyanidines);
carotenoids (for example lycopene, carotene);
proteins having an antioxidizing action (for example lactoferrin);
coenzymes (for example coenzyme Q10).

Other particularly useful bioactive substances are glycosaminoglycans, in particular hyaluronic acid.

Other bioactive substances that can be included in the compositions according to the present invention can be selected from: antibacterial agents, antiinflammatory agents and painkillers.

Said at least one bioactive substance can be included in the composition in quantities varying within wide limits, in relation to the specific substance, its activity and the effect to be obtained. Said at least one bioactive substance is generally present in the composition according to the present invention in a quantity ranging from 0.0001 to 30.0% by weight, preferably in a quantity ranging from 0.001 to 15.0% by weight, with respect to the overall weight of the composition.

According to a second aspect, the present invention relates to the use of a composition as defined above in formulations for cosmetic use.

According to a further aspect, the present invention relates to a first process for the preparation of a composition as defined above which comprises:

> providing a first aqueous suspension containing at least one PHA in the form of particles in suspension;
> providing a second aqueous suspension containing at least one calcium phosphate in the form of particles in suspension;
> mixing the first and second aqueous suspension, so as to obtain an aggregation between the PHA particles and the calcium phosphate particles.

According to a further aspect, the present invention relates to a second process for the preparation of a composition as defined above which comprises:

> providing a first aqueous solution comprising calcium ions with a concentration ranging from 0.1 moles/l to 10 moles/l, preferably ranging from 0.3 moles/l to 5 moles/l;
> adding, to said first aqueous solution, at least a first aqueous suspension of at least one PHA in the form of particles in suspension, so as to obtain a second suspension;
> adding, to said second suspension, a second aqueous solution containing phosphate ions at a concentration ranging from 0.1 moles/l to 10 moles/l, preferably ranging from 0.2 moles/l to 5 moles/l;
> maintaining the suspension thus obtained under stirring at a temperature ranging from 10°C to 80°C, preferably from 20°C to 50°C, so as to obtain an aggregation between the PHA particles and the calcium phosphate particles.

In both processes described above, at the end of the preparation, the composition according to the present invention can be separated as a precipitate, or the suspension can be used as such, particularly when the composition is intended for the preparation of formulations containing a high percentage of water.

As far as the PHA is concerned, this is used in the above processes in the form of particles in suspension, preferably having an average size ranging from 0.1 $\mu$m to 100 $\mu$m, more preferably from 0.2 $\mu$m to 20 $\mu$m.

For the preparation of the composition according to the present invention, it is advantageous to use the aqueous suspension of PHA obtained directly from the bacterial fermentation process which produces PHA itself, without having to precipitate it and dry it. The aqueous suspension obtained directly from the production process, in fact, has optimum characteristics in terms of homogeneity, dispersion and particle-size of PHA. The aqueous suspension of PHA obtained from the fermentation process is in any event preferably previously subjected to a purification and whitening step, so as to eliminate the residues and substances present in the fermentation broth.

With respect to the insertion of the bioactive substance, this can be added in any step of the preparation process of the composition. In the case of the first process according to the present invention described above, the bioactive substance is preferably added together with the calcium phosphate. This favours the bond between the bioactive substance and the calcium phosphate, which is then bound to the PHA.

Preferably, in the case of the second process according to the present invention described above, again with the purpose of favouring the interaction between the calcium phosphate and bioactive substance, the latter is added during the preparation of the calcium phosphate, in particular together with the solution of calcium ions or together with the solution of phosphate ions.

The following working examples are provided for purely illustrative purposes of the present invention and should not be considered as limiting the protection scope defined by the enclosed claims.

EXAMPLE 1

An aqueous suspension of hydroxyapatite (HA) was prepared as follows.

An aqueous solution of 25.0 g of $Ca(OH)_2$ and 0.2 g of $CaCO_3$ was prepared by mixing said products in 250 ml of demineralized water, kept under stirring for 1 hour at 45°C. A solution of 6.3 g of phosphoric acid ($H_3PO_4$) in 250 ml of demineralized water was added dropwise to this solution. The mixture thus obtained was kept under stirring for 24 hours at 25°C. The suspension of HA thus obtained was used as such as described hereunder.

The HA thus obtained was characterized by its crystallinity degree (CD), defined as above and measured in accordance with the method described in Landi, E., Tampieri, A., Celotti, G., Sprio, S., "Densification behaviour and mechanisms of synthetic hydroxyapatites", J. Eur. Ceram. Soc., 2000, 20, 2377-2387. The CD value obtained was equal to 71.0%.

A solution was prepared, consisting of 1 g of procyanidin dissolved in 7.5 g of demineralized water. The solution was added to the HA suspension prepared as described above (containing 10 g of HA). The dimensions of the single HA crystals ranged from 0.05 to 0.4 microns. These were in turn spontaneously aggregated in clusters having an average size of 1 micron.

The suspension was kept under stirring for about 60 minutes.

5 g of PHA, in the form of particles obtained from the fermentation broth without being dried, having an average size of around 10 $\mu$m, were suspended in 10 g of demineralized water, with vigorous stirring for 30 minutes (concentration of PHA: 10% by weight).

The suspension of HA containing procyanidin was mixed with the suspension of PHA, the whole mixture was kept under stirring for 120 minutes.

The composition according to the present invention was separated from the suspension thus obtained, by filtration.

The starting materials and the composition thus obtained were characterized as follows.

FT-IR spectra

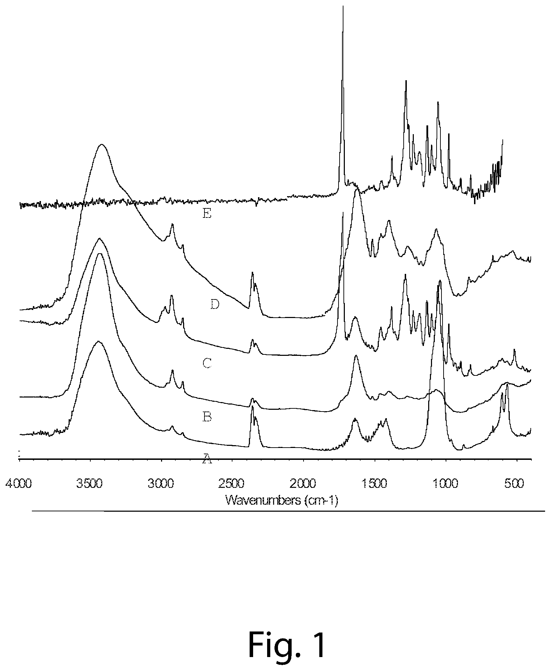

Figure 1 shows the FT-IR spectra of HA as such (spectrum A), of HA + procyanidin (spectrum B), of composition HA + PHA + procyanidin (spectrum C), of procyanidin as such (spectrum D), of PHA as such (spectrum E). In spectrum A relating to HA alone, the presence can be observed of the band typical of phosphate group at 1100-1030 cm$^{-1}$. After the combination with procyanidin (spectrum B), a remarkable reduction in the intensity of this band can be noted, and the accentuation of the bands at 2800-3000 cm$^{-1}$, characteristic of the groups present in procyanidin and visible in spectrum D.

After the combination with PHA (spectrum C), there is also the appearance of some bands typical of PHA, first of all that at 1800 cm$^{-1}$, clearly visible in the spectrum of PHA alone (spectrum E).

SEM images

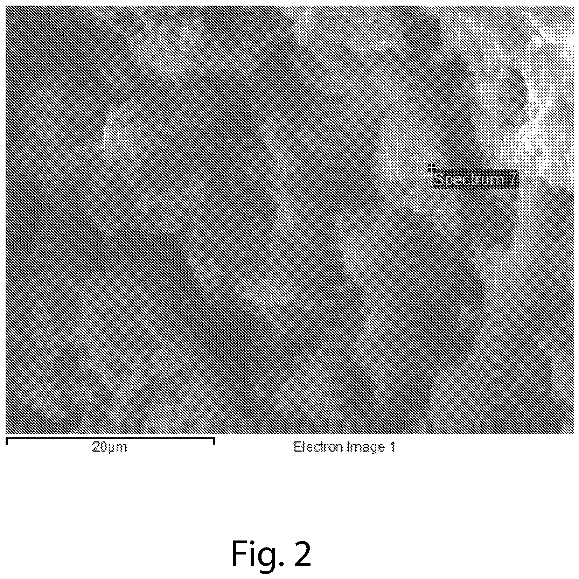

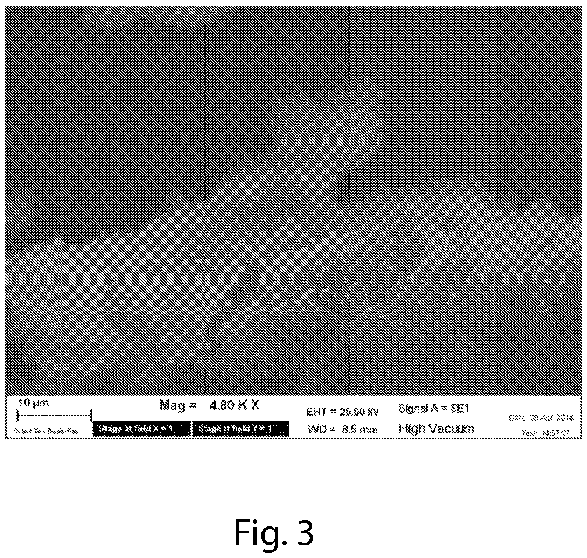

Some SEM (scanning electron microscope) images were taken of PHA particles as such (Figure 2) and PHA particles after being bound with the HA particles containing procyanidin (Figure 3). A different aggregation state of the PHA particles can be observed, before and after the combination with HA.

EDX analysis

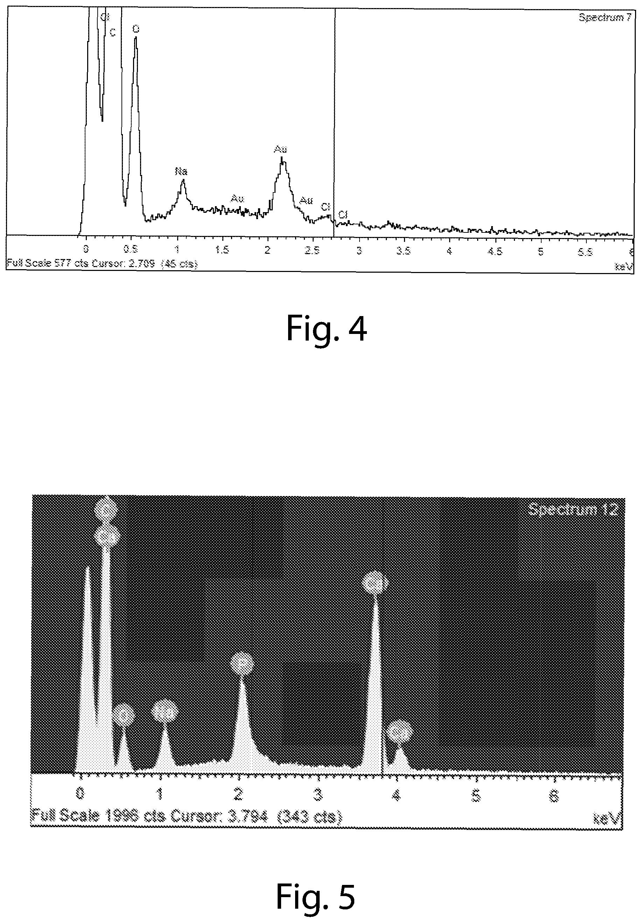

The composition of the surface of the particles of PHA as such (Figure 4) and of the particles of PHA after being bound to the HA particles containing procyanidin (Figure 5), was analyzed by means of elemental analysis with an EDX probe (Energy Dispersive X-ray Analysis).

Figure 4 shows how the EDX analysis carried out on the surface of the PHA particles reveals the presence of Au (gold), which is present as it is added by the instrument used for carrying out the analysis, sodium (probably present in the liquid in which the PHA is suspended), but above all carbon and oxygen.

In Figure 5, on the other hand, it can be noted that elemental analysis reveals the presence of calcium and phosphorous, elements characteristic of hydroxyapatite, and in the same ratio in which the latter was synthesized. This means that a thin layer of HA was deposited on the micrometric granules of PHA, which interacts with the HA.

EXAMPLE 2

A solution was prepared, consisting of 21 g of coenzyme Q10 dissolved in 7 g of demineralized water. The solution was added to a suspension in water of 10 g of hydroxyapatite (HA) obtained as described in Example 1.

The dimensions of the single crystals of HA ranged from 0.05 to 0.4 microns. These were in turn spontaneously aggregated in clusters having an average size of 1 micron.

The suspension was kept under stirring for about 60 min.

10 g of a suspension of PHA, obtained directly from the fermentation process for the production of PHA itself, previously subjected to purification, were mixed with the suspension of HA containing coenzyme Q10. The whole mixture was kept under stirring for 180 minutes.

The composition according to the present invention was separated from the suspension thus obtained, by filtration.

The starting materials and the composition thus obtained were characterized as follows.

FT-IR spectra

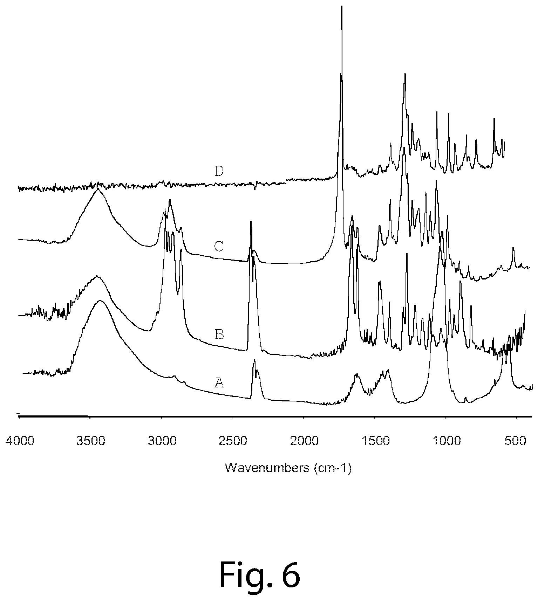

Figure 6 shows the FT-IR spectra of HA as such (spectrum A), of coenzyme Q10 as such (spectrum B), of composition HA + PHA + coenzyme Q10 (spectrum C), of PHA as such (spectrum D).

In spectrum A (relating to HA alone), the presence can be observed of the band typical of phosphate group at 1100-1030 cm$^{-1}$. After the combination between HA, Q10 and PHA (spectrum C), a significant reduction in the intensity of this band can be noted, and the accentuation of the bands at 2800-3000 cm$^{-1}$, characteristic of the groups present in Q10 and visible in spectrum B relating to Q10 alone. In spectrum C, there is also the appearance of some bands typical of PHA, first of all that at 1800 cm$^{-1}$, clearly visible in the spectrum of PHA alone (Spectrum D).

EXAMPLE 3

Example 2 was repeated using, instead of the solution of coenzyme Q10, a solution consisting of potassium ascorbate (0.20 g) dissolved in 5 ml of demineralized water.

FT-IR spectra

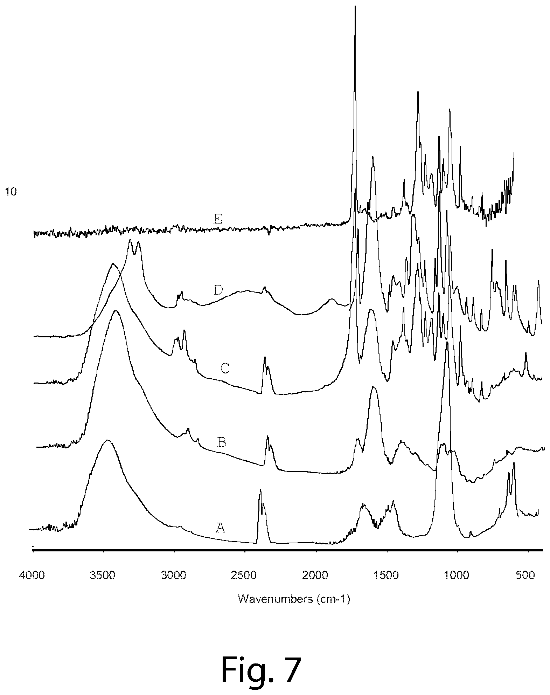

Figure 7 shows the FT-IR spectra of HA as such (spectrum A), of ascorbate + HA (spectrum B), of composition HA + PHA + ascorbate (spectrum C), of ascorbate as such (spectrum D), of PHA as such (spectrum E).

In spectrum A, the presence can be observed of the band typical of phosphate group at 1100-1030 cm$^{-1}$. After the combination with ascorbate (spectrum B), a significant reduction in the intensity of this band can be noted, and the accentuation of the bands at 2800-3000 cm$^{-1}$, characteristic of the groups present in the ascorbate and visible in spectrum D. Again in spectrum B, a broadening of the absorption band at 3200 cm$^{-1}$ can also be observed, due to the overtone of absorption bands typical of ascorbate with those of HA.

After the combination with PHA (spectrum C), in addition to the above-mentioned overtone at 3200 cm$^{-1}$, the appearance of some bands typical of PHA is noted, first of all that at 1800 cm$^{-1}$, clearly visible in the spectrum of PHA alone (spectrum E).

EXAMPLE 4

100 ml of a solution containing calcium ions with a concentration equal to 3.60 g/l were introduced into a reaction flask in an environment thermostat-regulated at 25°C.

10 g of a suspension of PHA, obtained directly from the fermentation process for the production of PHA itself, previously subjected to purification, were mixed with the above-mentioned solution of calcium ions. The whole mixture was kept under stirring for 60 minutes.

20 ml of an aqueous solution containing phosphate ions were prepared separately, at a concentration equal to 17.85 g/l. This solution was added dropwise (dripping rate equal to 0.3 ml/min), to the suspension of PHA containing calcium ions as indicated above, under vigorous stirring. The suspension was then subjected to maturation, keeping it under stirring at 25°C for about 24 hours.

Finally, a solution of lactoferrin, obtained by dissolving 200 mg of lactoferrin in 10 ml of demineralized water (concentration: 20 mg/ml), was added to the suspension. The whole mixture was kept under stirring for a time equal to about 360 minutes.

The composition according to the present invention was separated from the suspension thus obtained, by filtration. The starting materials and the composition thus obtained were characterized as follows.

FT-IR spectra.

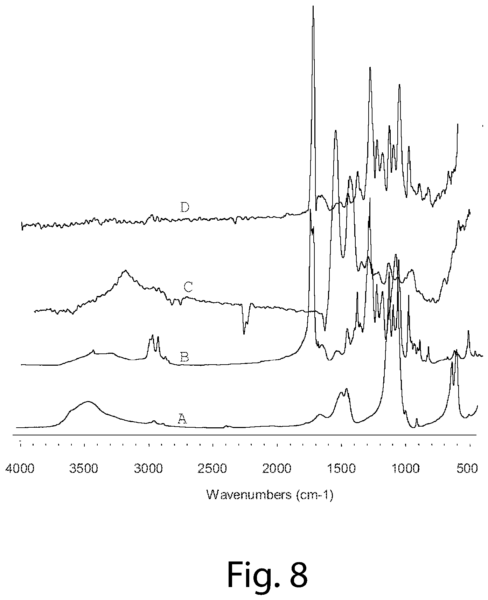

Figure 8 shows the FT-IR spectra of HA as such (spectrum A), of composition HA + PHA + lactoferrin (spectrum B), of lactoferrin as such (spectrum C), of PHA as such (spectrum D).

In spectrum A, the presence can be observed of the band typical of phosphate group at 1100-1030 cm$^{-1}$. After the combination with lactoferrin (spectrum B), a significant reduction in the intensity of this band can be noted, and the accentuation of the bands at 2800-3000 cm$^{-1}$, characteristic of the groups present in lactoferrin as such (spectrum C). Due to the interaction with PHA, in addition to the above-mentioned overtone at 3200 cm$^{-1}$, the appearance can also be noted of some bands typical of PHA, first of all that at 1800 cm$^{-1}$, clearly visible in the spectrum of PHA alone (Spectrum D).

EXAMPLE 5

Following the same procedure described in Example 4, two compositions of lactoferrin, hydroxyapatite and PHA were prepared, using solutions of lactoferrin with concentrations equal to 60 mg/ml and 80 mg/ml, and the same aqueous suspensions of HA and PHA used in Example 4 (weight ratio between lactoferrin and mixture of HA + PHA equal to 1:1). The antibacterial effectiveness of these compositions was assessed, by means of bacterial count on Petri plates with respect to the growth of *Streptococcus mutans* ATCC 35668. The results are reported in Table 1, which also shows for comparative purposes the percentage reduction values of bacterial growth for a mixture of HA and PHA (weight ratio 1:1), obtained from the same products used in Example 4, and for lactoferrin alone (aqueous solution with a concentration of 60 mg/ml and 80 mg/ml).

TABLE 1

|  | Reduction bacterial growth (%) |
|---|---|
| HA + PHA (1:1) | 8 |
| Lactoferrin (60 mg/ml) | 30 |
| Lactoferrin (80 mg/ml) | 35 |
| Lactoferrin (60 mg/ml) + HA + PHA | 41.8 |
| Lactoferrin (80 mg/ml) + HA + PHA | 62.2 |

From the results obtained, the improved antibacterial effect deriving from the use of the compositions according to the present invention with respect to the use of the separate components (synergic effect), is evident.

**Claims**

1. Composition comprising at least one polyhydroxyalkanoate (PHA) and at least one calcium phosphate, said at least one calcium phosphate being in the form of aggregates having an average size comprised between 0.1 $\mu$m and 10 $\mu$m, preferably between 0.2 $\mu$m and 5 $\mu$m, said micrometric aggregates being composed of calcium phosphate particles having average dimensions ranging from 0.01 $\mu$m to 1.0 $\mu$m and a crystallinity degree (CD) ranging from 50% to 80%, wherein the average size of the aggregates, the average dimensions of the calcium phosphate particles and the crystallinity degree are measured as indicated in the description.

2. Composition according to claim 1, wherein the crystallinity degree (CD) of the calcium phosphate particles ranges from 58% to 75%.

3. Composition according to claim 1 or 2, wherein said at least one PHA is a polymer containing repeating units of formula:

$$-O-CHR_1-(CH_2)_n-CO- \qquad (I)$$

wherein:

$R_1$ is selected from: -H, $C_1$-$C_{12}$ alkyls, $C_4$-$C_{16}$ cycloalkyls, $C_2$-$C_{12}$ alkenyls, possibly substituted by at least one group selected from: halogen (F, Cl, Br), -CN, -OH, - COOH, -OR, -COOR (R = $C_1$-$C_4$ alkyl, benzyl);
n is 0 or an integer from 1 to 6, preferably 1 or 2.

4. Composition according to any one of the preceding claims, wherein said at least one PHA has a weight average molecular weight ($M_w$) from 5,000 to 1,500,000 Da, preferably from 100,000 to 1,000,000 Da.

5. Composition according to any one of the preceding claims, wherein said at least one PHA is in the form of particles having an average size between 0.1 $\mu$m and 100 $\mu$m, preferably between 0.2 $\mu$m and 20 $\mu$m.

6. Composition according to any one of the preceding claims, wherein said at least one calcium phosphate is selected from: octacalcium phosphate, tricalcium phosphate, apatite, hydroxyapatite.

7. Composition according to any one of the preceding claims, wherein said at least one calcium phosphate is a hydroxyapatite of formula:

$$Ca_{10-x} Zn_x (PO_4)_{6-y}(CO_3)_{y+z}(OH)_{2-z}$$

wherein:

x is a number comprised between 0.0055 and 0.6;
y is a number comprised between 0.065 and 0.9; and
z is a number comprised between 0 and 0.32.

8. Composition according to any one of the preceding claims, wherein said at least one PHA is present in an amount from 60% to 98% by weight, preferably from 75% to 95% by weight, with respect to the total weight of the composition.

9. Composition according to any one of the preceding claims, wherein said at least one calcium phosphate is present in an amount from 2% to 40% by weight, preferably from 5 to 25% by weight, with respect to the total weight of the composition.

10. Composition according to any one of the preceding claims, further comprising at least one bioactive substance.

11. Composition according to claim 10, wherein said at least one bioactive substance is selected from: antioxidants, antibacterial substances, antiinflammatories and painkillers.

12. Use of a composition according to any one of claims from 1 to 11, in formulations for cosmetic use.

13. Process for preparing a composition according to any one of the claims from 1 to 11, comprising:

providing a first aqueous suspension containing at least one PHA in the form of particles in suspension;
providing a second aqueous suspension containing at least one calcium phosphate in the form of particles in suspension;
mixing the first and the second aqueous suspension, so as to obtain an aggregation between PHA particles and calcium phosphate particles.

14. Process according to claim 13, wherein the second aqueous suspension containing at least one calcium phosphate is obtained by adding an aqueous solution of phosphoric acid ($H_3PO_4$) to an aqueous solution containing calcium ions ($Ca^{2+}$), and mixing the solution thus obtained until the aqueous suspension of calcium phosphate is obtained.

15. Process for preparing a composition according to any one of the claims from 1 to 11, comprising:

providing a first aqueous solution comprising calcium ions with a concentration comprised between 0.1 moles/l and 10 moles/l, preferably between 0.3 moles/l and 5 moles/l;
adding to said first aqueous solution at least one first aqueous suspension of at least one PHA in the form of particles in suspension, so as to obtain a second suspension;
adding to said second suspension a second aqueous solution containing phosphate ions at a concentration comprised between 0.1 moles/l and 10 moles/l, preferably between 0.2 moles/l and 5 moles/l;
maintaining the thus obtained suspension under stirring at a temperature comprised between 10°C and 80°C, preferably between 20°C and 50°C, so as to obtain an aggregation between PHA particles and calcium phosphate particles.

**Patentansprüche**

1. Zusammensetzung, umfassend mindestens ein Polyhydroxyalkanoat (PHA) und mindestens ein Calciumphosphat, wobei das mindestens eine Calciumphosphat in Form von Aggregaten mit einer mittleren Größe zwischen 0.1 $\mu$m und 10 $\mu$m, vorzugsweise zwischen 0.2 $\mu$m und 5 $\mu$m, vorliegt, wobei die mikrometrischen Aggregate aus Calciumphosphatpartikeln mit mittleren Abmessungen zwischen 0.01 $\mu$m bis 1.0 $\mu$m und einem Kristallinitätsgrad (CD) im Bereich von 50% bis 80% bestehen, wobei die durchschnittliche Größe der Aggregate, die durchschnittlichen Abmessungen der Calciumphosphatpartikel und der Kristallinitätsgrad wie in der Beschreibung angegeben gemessen werden.

2. Zusammensetzung nach Anspruch 1, wobei der Kristallinitätsgrad (CD) der Calciumphosphatpartikel im Bereich von 58% bis 75% liegt.

3. Zusammensetzung nach Anspruch 1 oder 2, wobei das mindestens eine PHA ein Polymer ist, das sich wiederholende Einheiten der Formel enthält:

$$-O-CHR_1-(CH_2)_n-CO- \qquad (I)$$

wobei:

R$_1$ ausgewählt ist aus: -H, C$_1$-C$_{12}$-Alkylen, C$_4$-C$_{16}$-Cycloalkylen, C$_2$-C$_{12}$-Alkenylen, gegebenenfalls substituiert durch mindestens eine Gruppe, ausgewählt aus: Halogen (F, C1, Br), -CN, -OH, -COOH, -OR, -COOR (R = C$_1$-C$_4$-Alkyl, Benzyl);
n gleich 0 oder eine ganze Zahl von 1 bis 6, vorzugsweise 1 oder 2, ist.

4. Zusammensetzung nach einem der vorstehenden Ansprüche, wobei das mindestens eine PHA ein gewichtsmittleres Molekulargewicht (Mw) von 5,000 bis 1,500,000 Da, vorzugsweise von 100,000 bis 1,000,000 Da, aufweist.

5. Zusammensetzung nach einem der vorstehenden Ansprüche, wobei das mindestens eine PHA in Form von Partikeln mit einer durchschnittlichen Größe zwischen 0.1 $\mu$m und 100 $\mu$m, vorzugsweise zwischen 0.2 $\mu$m und 20 $\mu$m, vorliegt.

6. Zusammensetzung nach einem der vorstehenden Ansprüche, wobei das mindestens eine Calciumphosphat ausgewählt ist aus: Octacalciumphosphat, Tricalciumphosphat, Apatit, Hydroxyapatit.

7. Zusammensetzung nach einem der vorstehenden Ansprüche, wobei das mindestens eine Calciumphosphat ein Hydroxyapatit der Formel ist:

$$Ca_{10-x}Zn_x(PO_4)_{6-y}(CO_3)_{y+z}(OH)_{2-z}$$

wobei:

x eine Zahl ist, die zwischen 0.0055 und 0.6 liegt;
y eine Zahl ist, die zwischen 0.065 und 0.9 liegt; und
z eine Zahl ist, die zwischen 0 und 0.32 liegt.

8. Zusammensetzung nach einem der vorstehenden Ansprüche, wobei das mindestens eine PHA in einer Menge von 60 Gew.-% bis 98 Gew.-%, vorzugsweise von 75 Gew.-% bis 95 Gew.-%, bezogen auf das Gesamtgewicht der Zusammensetzung, vorliegt.

9. Zusammensetzung nach einem der vorstehenden Ansprüche, wobei das mindestens eine Calciumphosphat in einer Menge von 2 Gew.-% bis 40 Gew.-%, vorzugsweise von 5 Gew.-% bis 25 Gew.-%, bezogen auf das Gesamtgewicht der Zusammensetzung, vorliegt.

10. Zusammensetzung nach einem der vorstehenden Ansprüche, ferner umfassend mindestens eine bioaktive Substanz.

11. Zusammensetzung nach Anspruch 10, wobei die mindestens eine bioaktive Substanz ausgewählt ist aus: Antioxidantien, antibakteriellen Substanzen, Entzündungshemmern und Schmerzmitteln.

12. Verwendung einer Zusammensetzung nach einem der Ansprüche 1 bis 11 in Formulierungen zur kosmetischen Verwendung.

13. Verfahren zur Herstellung einer Zusammensetzung nach einem der Ansprüche 1 bis 11, umfassend:

Bereitstellen einer ersten wässrigen Suspension, die mindestens ein PHA in Form von Partikeln in Suspension enthält;

Bereitstellen einer zweiten wässrigen Suspension, die mindestens ein Calciumphosphat in Form von Partikeln in Suspension enthält;

Mischen der ersten und der zweiten wässrigen Suspension, um eine Aggregation zwischen PHA-Partikeln und Calciumphosphatpartikeln zu erhalten.

14. Verfahren nach Anspruch 13, wobei die zweite wässrige Suspension, die mindestens ein Calciumphosphat enthält, durch Zugabe einer wässrigen Lösung von Phosphorsäure ($H_3PO_4$) zu einer wässrigen Lösung, die Calciumionen ($Ca^{2+}$) enthält, und Mischen der so erhaltenen Lösung erhalten wird, bis die wässrige Suspension von Calciumphosphat erhalten wird.

15. Verfahren zur Herstellung einer Zusammensetzung nach einem der Ansprüche 1 bis 11, umfassend:

Bereitstellen einer ersten wässrigen Lösung, die Calciumionen in einer Konzentration zwischen 0.1 Mol/1 und 10 Mol/1, vorzugsweise zwischen 0.3 Mol/1 und 5 Mol/1, umfasst;

Zugeben mindestens einer ersten wässrigen Suspension von mindestens einem PHA in Form von Partikeln in Suspension zu der ersten wässrigen Lösung, um eine zweite Suspension zu erhalten;

Zugeben einer zweiten wässrigen Lösung zu der zweiten Suspension, die Phosphationen in einer Konzentration zwischen 0,1 Mol/1 und 10 Mol/1, vorzugsweise zwischen 0.2 Mol/1 und 5 Mol/1, enthält;

Halten der so erhaltenen Suspension unter Rühren auf einer Temperatur zwischen 10°C und 80°C, vorzugsweise zwischen 20°C und 50°C, um eine Aggregation zwischen PHA-Partikeln und Calciumphosphatpartikeln zu erreichen.

## Revendications

1. Composition comprenant au moins un polyhydroxyalcanoate (PHA) et au moins un phosphate de calcium, ledit au moins un phosphate de calcium étant sous la forme d'agrégats ayant une taille moyenne comprise entre 0.1 $\mu$m et 10 $\mu$m, de préférence entre 0.2 $\mu$m et 5 $\mu$m, lesdits agrégats micrométriques étant composés de particules de phosphate de calcium ayant des dimensions moyennes allant de 0.01 $\mu$m à 1.0 $\mu$m et un degré de cristallinité (CD) allant de 50% à 80%, dans lequel la taille moyenne des agrégats, les dimensions moyennes des particules de phosphate de calcium et le degré de cristallinité sont mesurés comme indiqué dans la description.

2. Composition selon la revendication 1, dans laquelle le degré de cristallinité (CD) des particules de phosphate de calcium va de 58% à 75%.

3. Composition selon la revendication 1 ou 2, dans laquelle ledit au moins un PHA est un polymère contenant des unités répétitives de formule:

$$-O\text{-}CHR_1\text{-}(CH_2)_n\text{-}CO- \qquad (I)$$

dans laquelle:

$R_1$ est sélectionné parmi: -H, alkyles en $C_1$-$C_{12}$, cycloalkyles en $C_4$-$C_{16}$, alcényles en $C_2$-$C_{12}$, possiblement substitués par au moins un groupe sélectionné parmi: un halogène (F, C1, Br), -CN, -OH, -COOH, -OR, -COOR (R = alkyle en $C_1$-$C_4$, benzyle);

n est 0 ou un nombre entier de 1 à 6, de préférence 1 ou 2.

4. Composition selon l'une quelconque des revendications précédentes, dans laquelle ledit au moins un PHA a une masse moléculaire (Mw) moyenne en poids de 5,000 à 1,500,000 Da, de préférence de 100,000 à 1,000,000 Da.

5. Composition selon l'une quelconque des revendications précédentes, dans laquelle ledit au moins un PHA est sous la forme de particules ayant une taille moyenne entre 0.1 $\mu$m et 100 $\mu$m, de préférence entre 0.2 $\mu$m et 20 $\mu$m.

6. Composition selon l'une quelconque des revendications précédentes, dans laquelle ledit au moins un phosphate de calcium est sélectionné parmi : le phosphate octacalcique, le phosphate tricalcique, l'apatite, l'hydroxyapatite.

7. Composition selon l'une quelconque des revendications précédentes, dans laquelle ledit au moins un phosphate de calcium est une hydroxyapatite de formule :

$$Ca_{10-x} Zn_x(PO_4)_{6-y}(CO_3)_{y+z}(OH)_{2-z}$$

dans laquelle:

x est un nombre compris entre 0.0055 et 0.6;
y est un nombre compris entre 0.065 et 0.9; et
z est un nombre compris entre 0 et 0.32.

8. Composition selon l'une quelconque des revendications précédentes, dans laquelle ledit au moins un PHA est présent en une quantité de 60% à 98% en poids, de préférence de 75% à 95% en poids, par rapport au poids total de la composition.

9. Composition selon l'une quelconque des revendications précédentes, dans laquelle ledit au moins un phosphate de calcium est présent en une quantité de 2% à 40% en poids, de préférence de 5 à 25% en poids, par rapport au poids total de la composition.

10. Composition selon l'une quelconque des revendications précédentes, comprenant en outre au moins une substance bioactive.

11. Composition selon la revendication 10, dans laquelle ladite au moins une substance bioactive est sélectionnée parmi: les antioxydants, les substances antibactériennes, les anti-inflammatoires et les analgésiques.

12. Utilisation d'une composition selon l'une quelconque des revendications de 1 à 11, dans des formulations pour une utilisation cosmétique.

13. Procédé pour préparer une composition selon l'une quelconque des revendications de 1 à 11, comprenant:

fournir une première suspension aqueuse contenant au moins un PHA sous la forme de particules en suspension;
fournir une seconde suspension aqueuse contenant au moins un phosphate de calcium sous la forme de particules en suspension;
mélanger la première et la seconde suspension aqueuse, de manière à obtenir une agrégation entre les particules de PHA et les particules de phosphate de calcium.

14. Procédé selon la revendication 13, dans lequel la seconde suspension aqueuse contenant au moins un phosphate de calcium est obtenue en ajoutant une solution aqueuse d'acide phosphorique ($H_3PO_4$) à une solution aqueuse contenant des ions calcium ($Ca^{2+}$), et en mélangeant la solution ainsi obtenue jusqu'à obtenir la suspension aqueuse de phosphate de calcium.

15. Procédé pour préparer une composition selon l'une quelconque des revendications de 1 à 11, comprenant:

fournir une première solution aqueuse comprenant des ions calcium avec une concentration comprise entre 0.1 moles/l et 10 moles/1, de préférence entre 0.3 moles/1 et 5 moles/1 ;
ajouter à ladite première solution aqueuse au moins une première suspension aqueuse d'au moins un PHA sous la forme de particules en suspension, de manière à obtenir une seconde suspension;
ajouter à ladite seconde suspension une seconde solution aqueuse contenant des ions phosphate à une concentration comprise entre 0.1 moles/1 et 10 moles/1, de préférence entre 0.2 moles/1 et 5 moles/l ;
maintenir la suspension ainsi obtenue sous agitation à une température comprise entre 10°C et 80°C, de préférence entre 20°C et 50°C, de manière à obtenir une agrégation entre les particules de PHA et les particules de phosphate de calcium.

# Fig. 1

20μm        Electron Image 1

# Fig. 2

Fig. 3

Fig. 4

Fig. 5

Fig. 6

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9923146 A
- WO 2011045625 A
- WO 2015015315 A
- US 8956835 B2

**Non-patent literature cited in the description**

- **LANDI, E. ; TAMPIERI, A. ; CELOTTI, G. ; SPRIO, S.** Densification behaviour and mechanisms of synthetic hydroxyapatites. *J. Eur. Ceram. Soc.,* 2000, vol. 20, 2377-2387
- **Y. WANG et al.** *Materials Letters,* 2007, vol. 61, 1071-1076